# EUROPEAN PATENT APPLICATION

(11) **EP 0 911 634 A1**
(43) Date of publication of application: **28.04.1999**
(21) Application number: 97203296.5
(22) Date of filing: 24.10.1997
(51) Int. Cl.: G01N 33/573, A61K 38/00, A61K 31/52

(54) **Pharmaceutical uses of CDK-2 regulators**

(71) Applicant: Het Nederlands Kanker Instituut, 1066 CX Amsterdam (NL)
(72) Inventor: The designation of the inventor has not yet been filed
(74) Representative: Smulders, Theodorus A.H.J., Ir.

(57) **Abstract**

The invention relates to substances interfering with pathways of programmed cell death and/or cell division.

The invention provides a method for inhibiting apoptotic processes in a cell comprising providing said cell with at least one inhibitor of cyclin-dependent kinase 2 or an inhibitor of a functional equivalent of cyclin-dependent kinase 2. One such an inhibitor is roscovitine or a functional equivalent thereof. The invention also provides the sue of such an inhibitor in the treatment of apoptosis, such as observed in diseases associated with apoptosis or with transplants.

## Description

The present invention relates to substances interfering with pathways of programmed cell death and/or cell division.

Programmed cell death, also known as apoptosis, is a ubiquitous physiological process used to eliminate damaged or unwanted cells in multicellular organisms. For example, apoptosis serves to ensure the selection of appropriate lymphoid populations during T cell development in the thymus. Disregulation of apoptosis is now widely believed to be involved in the pathogenesis of many human diseases. The failure of apoptotic cell death has been implicated in various cancers, as well as autoimmune disorders. Conversely, increased apoptosis is associated with a variety of diseases involving cell loss such as neurodegenerative disorders and AIDS. The process of apoptosis is an active one and regulated by certain gene products which are conserved from nematodes to mammals and either block or accelerate programmed cell death. Like apoptosis, cell division is a fundamental and ubiquitous process in multicellular organisms. Unlike apoptosis, much is known about the components of cell cycle progression and mitosis such as the cyclins and cyclin dependent kinases (Cdks). Evidence exists to suggest that the cell cycle and apoptosis may be interconnected. For example, expression of the proto-oncogene c-myc stimulates cell proliferation and can also predispose cells to apoptosis when growth factors are limiting. More recent work now indicates that the apoptosis regulatory proteins themselves can directly impinge on the cell cycle machinery.

Bax and Bcl-2 are two of the gene products that can regulate apoptosis. Bax overexpression has been shown to accelerate cell death in response to certain apoptotic stimuli such as growth factor withdrawal, γ-irradiation or glucocorticoid treatments whereas Bcl-2 can inhibit cell death in such circumstances. Bax and Bcl-2 can form heterodimers and overexpression of one antagonises the other's effect. They are part of two classes of Bcl-2 related proteins. One class includes Bcl-2 and Bcl-X_{L} which can inhibit apoptosis, the other class includes proteins such as Bax, Bak and Bcl-Xₛ which promote apoptosis. No biochemical mechanism has so far been delineated which can account for the effects of Bcl-2 and Bax.

We and others have demonstrated that overexpression of the apoptosis regulatory proteins Baxα and Bcl-2 in T cells of transgenic mice leads to perturbations in the dividing population of thymocytes. The effect of *baxα* is to increase the number of cycling thymocytes whereas *bcl*-2 has the opposite effect. Furthermore, by studying the IL-2 mediated transition from G₁ to S phase of activated T cells from transgenic mice *bcl-2* overexpression has been shown to prolong the period before entry into S phase whereas *bax* foreshortens it. This correlates with the levels of P27^{KiP1} in the activated T cells since *bcl-2* overexpression is seen to delay the degradation of p27^{Kip1} whereas *bax* accelerates it. p27^{KiPl} is a negative regulator of Cdk activity. The Cdks in turn are positively regulated by association with cyclins and are required for cell cycle progression. Since p27^{KiP1} has been demonstrated to be a major inhibitor for Cdk2, we examined whether Bax and Bcl-2 could modulate Cdk2 activity during thymocyte apoptosis.

Since apoptosis and cell division are both essential processes in the pathogenesis of many diseases and seem to be highly interconnected, it is of crucial importance that methods and means are provided to direct cells to either pathway without inducing cells to enter the other pathway and/or to inhibit either pathway without inhibiting the other pathway. The present invention, using thymocytes as an example now provides the identification of a key regulatory molecule, which stands at the crossroads between apoptosis and cell division and thereby the present invention provides methods and means to direct cells to a certain pathway or to prevent cells from entering a certain pathway. Since both pathways are conserved throughout multicellular organisms, the example of mouse thymocytes can be applied to many other cell types and organisms.

The key molecule identified in the present invention is cyclin-dependent kinase 2 (also referred to herein as CDK-2). Thus the invention provides a method for inhibiting apoptotic processes in a cell comprising providing said cell with at least one inhibitor of cyclin-dependent kinase 2 or an inhibitor of a functional equivalent of cyclin-dependent kinase 2.

In fact it is only required that the activity of cyclin-dependent kinase 2 is inhibited, be it through a direct inhibitor or any other means. Such inhibition can be achieved by a variety of means, for example by antisense approach, or by promoting CDK2 degradation, or by inhibiting CDK2 activation by modulating post-translational modifications such as inhibiting activating phosphorylation by CAK(s), inhibiting activating the dephosphorylation by cdc25A, or than inacting phosphorylation by activating weel, or inactivating dephosphorylation. Yet another method to inhibit CDK2 can be achieved by enhancing negative regulation of CDK2 by inhibitors such as p27^{Kip1}. Such inhibition can be achieved by promoting the synthesis of P27^{Kip1} and similar proteins, by inhibiting their degradation, by for example using proteasome inhibitors and tetrapeptide aldehydes, by inhibiting enzymes responsible for targetting the inhibitors to the degradative pathway, for example inhibitors as E₁, E₂ and E₃ enzymes that transfer ubiquitine molecules to p27^{Kip1}. Or, in general, by inhibiting degenerative pathways of p27^{KipI}, or by inhibiting ubiquitine pathways.

Also modulating post translational modifications, and means such as mimicking peptides and peptides or antibodies capable of stabilising the CDK inhibitor CDK interaction may be used. Thus the invention also provides a method for inhibiting apoptotic processes in a cell comprising inhibiting cyclin-dependent kinase 2 activity or a functional equivalent of cyclin-dependent kinase 2. Many cells may be the target of a pharmaceutical intervention intended to either prevent apoptosis or to induce apoptosis. Preventing apoptosis in thymocytes and/or T-cells is an exemplified embodiment of the present invention. It is also a preferred embodiment because these cells and their progeny are important effectors of the immune response.

Other interesting targets are for instance cells which are subject to accelerated apoptosis such as in neurodegenerative diseases which may lead to loss of post mitotic neurones, or cells which are subject to apoptosis following reperfusion injury or cells in which apoptosis is induced by HIV (T cells).

Another important target population of cells is a subset of cells which is subject to apoptosis following trauma like heart attacks, seizures, brain infarcts and the like whereby it is very important to prevent massive cell death.

In this way, the acute and immediate or short term damage occurring after such sudden events can be limited, thereby preventing further damage to surrounding tissues and cells, for example of neuronal origin. Yet another important target population of cells and/or tissues (and even whole organs) are those living cells, tissues, organs and/or organ systems that are intended to be used for transplantation purposes. Such cells or tissues can for example be Langerhans cells, or hepatocytes, or bone marrow, or pancreatic islet cells or (parts of) whole organs such as kidney, lung, liver, or embryos, or germline cells, or stemcells, or oocytes or spermcells, but many other transplants are currently being used which can now be treated according to a method provided by the invention with an inhibitor of the apoptotic pathway, thereby extending or improving the life-time and/or viability of the transplant. By treating such intended transplants (that are possible taken out of the body of a already deceased donor, but that can also be derived from living sources) with an inhibitor of the apoptotic pathway, as provided by the invention, it is now possibly to prolong the viability of such transplants (made up of transplant cells), thereby facilitating better and more use of such transplants, which for example arrive, and can be transplanted in the receptor, in a better condition, with the pathway of cell death in the transplant cells arrested by the action of an inhibitor provided by the invention. The invention also provides a compound for treating a transplant, comprising an inhibitor provided by the invention. Such a compound generally meets pharmaceutical standards of quality, safety and sterility. The transplants can for instance be pre-treated, stored and/or transported in a compound, such as medium containing said inhibitor, or they can be perfused with a compound, such as a buffer solution containing an inhibitor provided by the invention. The invention provides transplants treated with an inhibitor provided by the invention. Such transplants provided by the invention, with for example an extended lifetime, can be obtained by treating them according to a method provided by the invention with a compound inhibiting the apoptotic pathway.

A very suitable way to arrive at inhibition of CDK-2 and thus of apoptosis is to provide the target cells to be prevented from entering the apoptotic pathway with an inhibitor of CDK-2. The present invention exemplifies such an inhibitor. The exemplified inhibitor is roscovitine (2-(1-D,L-hydroxymethylpropylamino)-6-benzylamino-9- isopropylpurine). Of course the invention is not limited to this specific preferred inhibitor. It at least includes functional equivalents of roscovitine. Functional equivalents for the present invention are defined as molecules having similarities with the original molecule such as exhibiting at least the capability to inhibit CDK-2 activity (in kind, not necessarily in amount). For the person skilled in the art it will be possible to modify the structure of roscovitine without substantially altering its kind of activity, though the amount may vary. Typical so-called conventional modifications are well known in the art. An attractive candidate functional equivalent of roscovitine is for example olomoucine, or flavopyridol, or butyrolacton-1, or HD, which are all functionally related molecules known to inhibit cyclin dependent kinases, or a molecule obtainable by testing compounds derived from a (chemical) combinatorial library for interaction with apoptosis mediated via CDK-2.

However, completely different inhibitors of CDK-2 are also within reach of the person skilled in the art. For instance, as CDK-2 has its apoptosis inducing activity under the influence of cyclins, it will be feasible to arrive at an antagonistic cyclin, which for instance does bind to CDK-2, but does not induce the apoptotic activity. Another inhibitor that is directly obtainable for persons skilled in the art is an antibody or a fragment or a derivative thereof or a mimicking peptide (i.e. a functional equivalent of an antibody) directed against the center of activity of CDK-2 or an epitope in the vicinity thereof, or such an antibody or functional equivalent thereof which binds to or near the cyclin binding site. Other substances than antibodies effectively blocking (or interfering with) the above-mentioned sites are of course equally suitable. Another clear possibility is blocking the ATP-binding site of CDK-2 as roscovitine does. This application is directed specifically to CDK-2, as being a key molecule at the intersection of the apoptotic pathways and cell cycle pathways. It may be the case that in some cells there is a functional equivalent of CDK-2, located at this very important intersection. Now that the present invention provides the knowledge that such a molecule at the mentioned intersection exists, it will be possible to identify said functional equivalent of CDK-2 (if it exists) in other cells. It is thought that such a molecule is within the scope of the present invention. In another aspect of the present invention, it is now possible to stop apoptosis in a certain subset of cells, if a targetting moiety associated with said subset is available. In that event it is easily feasible to make a conjugate of the inhibitor of CDK-2 and the targetting moiety. It is of course preferred that the targetting moiety is directed to a target molecule associated with the surface of the target cells and that the complex of target molecule and conjugate is internalized upon binding or shortly after. Typical targetting moieties include antibodies or functional equivalents thereof and ligands for receptors on cell surfaces. The more specific the targetting moiety is for the target the better, although complete specificity for a certain subset of cells seems very hard to achieve. It is therefore that concepts like prodrug activation and the like have been developed for especially targetted therapy regimes. Such prodrug approaches and other means for realizing a good localization of the therapeutic agent at the target site are of course preferred for the present invention as well. An important target for the approach mentioned above is for instance inhibition of apoptosis in Alzheimer's disease, Parkinson's syndrome or in other neurodegenerative disorders by preventing apopotosis in cells of neuronal origin.

In the same fashion as CDK-2 can be positively regulated by association with cyclins (such as cyclin E and A) in activating the cell cycle, CDK-2 can associate with and be regulated by another molecule with a name such as roscocyclin or cyclin X or apoptotic cyclin in activating apoptotic activity. Inhibition of such apoptotic cyclin can be achieved by a variety of means, for example by antisense approach, by promoting their degradation, by inhibiting their activation by modulating their post translational modifications, by use of antibodies, fragments thereof or mimicking peptides, or by using peptides capable to block the cyclin-CDK interaction.

The beauty of CDK-2 being a key molecule in the apoptosis pathway is of course that it can also be used to induce apoptosis. Induction of apoptosis is of course a major tool in destroying subsets of cells in an organism. This means that for instance tumor cells can be contacted with a means to enhance CDK-2 activity, leading the tumor cells down the apoptotic pathway. Great care of course has to be taken that cells are not induced to enter the other route controlled by CDK-2, i.e. the cell cycle. Again it is preferred that CDK-2 activity to induce apoptosis is delivered only to the subset of cells to be eliminated, therefore conjugates of targetting moieties and molecules delivering CDK-2 apoptotic activity are preferred.

Another subset of cells that is an important target for elimination in certain pathologies is the subset T-cells.

In treatment of transplantation rejection or GvH disease it is often desired to eliminate a certain group of T-cells. Another approach is to effect the activity of the above mentioned roscocyclin.

The invention will now be explained in more detail in the following experimental part.

### Experimental.

### Methods

### Apoptosis Assay

The assays measuring the percentage of apoptotic thymocytes were performed as described previously.

Thymocytes were resuspended in a hypotonic buffer containing 50u(micro)g/ml propidium iodide followed by flow cytometry analysis to determine the percentage of cells containing hypodiploid DNA that indicate apoptosis.

The antiFas antibody was Jo2 (Pharmingen). Roscovitine was dissolved in DMSO prior to use and was a gift from Dr. Laurent Meijer, CNRS, Roscoff, France. The proteasome inhibitor Cbz-LLL was a gift from Prof. Hidde Ploegh, M.I.T., Boston and LLNL was purchased from Boehringer Mannheim.

### Immunoblotting

Cell lysates (5 x 10⁵ cells per lane) were immunoblotted using enhanced chemiluminescence (Amersham) for detection The anti P27^{KiP1} (C- 19) and anti Cdk2 (M2) antibodies were purchased from Santa Cruz Biotech and the pan-actin antibody (C4) from Boehringer Mannheim.

### Kinase assays

For the determination of kinase activity, an equivalent number of thymocytes (5 x 10⁵ viable cells at time zero) were pelleted and stored at -80°C until assayed. Kinase activity was assayed by immunoprecipitation of the kinase followed by incubation with a substrate, histone H1, and γ³²P ATP. The immunoprecipitations and histone H1 kinase assays were performed exactly as described (Kranenburg et al., Oncogene 10, 87-95, 1995). The M2 antibody (Santa Cruz) was used for Cdk2 and the A 17 antibody (Pharmingen) for Cdkl immunoprecipitations. The kinase assays were resolved on a 12% SDS polyacrylamide gel, stained with Coomassie Blue to visualise the histone H1 bands, dried and exposed for autoradiography. In some experiments, the histone H1 bands were excised and counted on a liquid scintillation beta counter. Cyclin E and A associated kinase activity was assessed following inununoprecipitation with either the H-432 or C- 19 antibody for cyclin A and M-20 antibody for cyclin E (au from Santa Cruz Biotech).

### Cdk2 kinase activity rises during thymocyte apoptosis

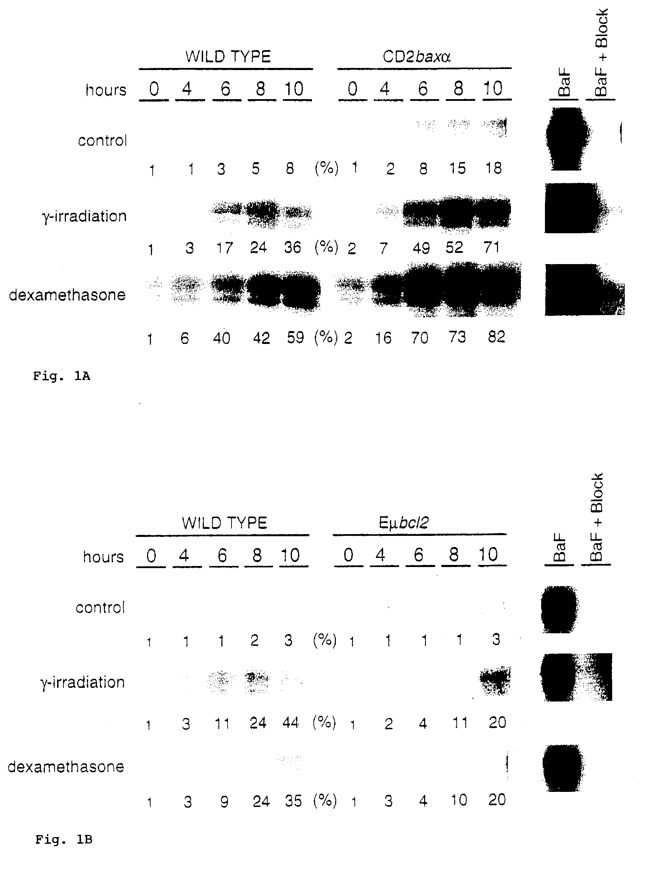

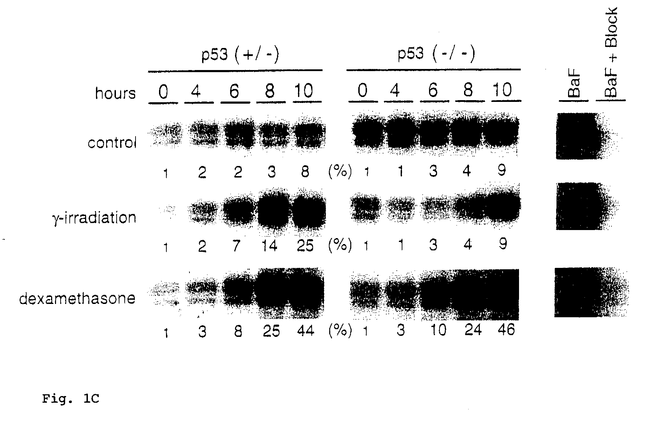

We removed thymocytes from wild type and transgenic mice and cultured them *in vitro* either in medium alone or following γ-irradiation (p53-dependent apoptosis) or incubation with the glucocorticoid dexamethasone (p53-independent apoptosis). To determine the level of its activity, Cdk2 is first immunoprecipitated from equivalent numbers of thymocytes and then co-incubated with histone H1, to act as a substrate and [γ-³²P]ATP. The Cdk2 phosphorylates the histone H1 and the amount of 32P incorporated into the histone gives a measure of Cdk2 kinase activity. The level of phosphorylation of histone H1 after immunoprecipitation of Cdk2 from the dying thymocytes increased with time either in culture alone or more dramatically following apoptotic stimulus. The percentage of apoptotic cells present during the process was determined using a flow cytometry-based technique which measures propidium iodide staining of DNA after cell lysis in a hypotonic buffer. This showed that the elevation in Cdk2 kinase activity correlated with the appearance of apoptotic cells for all the stimuli tested (Fig. 1 a, b and c). As we have reported previously, thymocytes from *baxα* transgenic mice are more susceptible to die after triggering them with p53-dependent and independent apoptotic stimuli. Measurement of the Cdk2 kinase activity in the conditions described above showed that Bax*α* accelerated apoptosis correlated with higher levels of Cdk2 kinase activity (Fig. 1a). Conversely, overexpression of the survival gene *bcl-2* in transgenic thymocytes leads to resistance to apoptotic stimuli and to lower levels of Cdk2 activity (Fig. lb).

In order to further validate the correlation between Cdk2 activation and the appearance of apoptosis, we carried out similar experiments as described above using instead thymocytes from p53(+/-) and p53(-/-) mice.

Thymocytes from p53(-/-) mice are highly resistant to genotoxic agents like γ-radiation and chemotherapeutic drugs, whereas they remain fully sensitive to dexamethasone induced apoptosis. There is a time-dependent increase in Cdk2 kinase activity in thymocytes from p53(+/-) mice triggered to die by γ-radiation or dexamethasone treatment which correlates with the number of apoptotic cells (Fig. Ic). As expected, there is no difference in the susceptibility of either p53(-/-) or p53(+/-) thymocytes to dexamethasone induced cell death in terms of cells undergoing apoptosis and this is reflected in the levels and kinetics of Cdk2 induction.

Whereas, p53 (+/-) thymocytes behave comparably to wild type thymocytes in γ-radiation induced apoptosis, p53(-/-) thymocytes do not show increases in either cell death or Cdk2 kinase activity during the same time span (Fig. 1c). Cdk2 activity does begin to appear by the 10 h time point of p53(-/-) γ-irradiated thymocytes as by then even they are beginning to apoptose. From these results we conclude that there is a correlation between the levels of p53 dependent or independent apoptosis in thymocytes and the activation of Cdk2 kinase activity.

### Cdk2 kinase activity is obligatory for thymocyte apoptosis

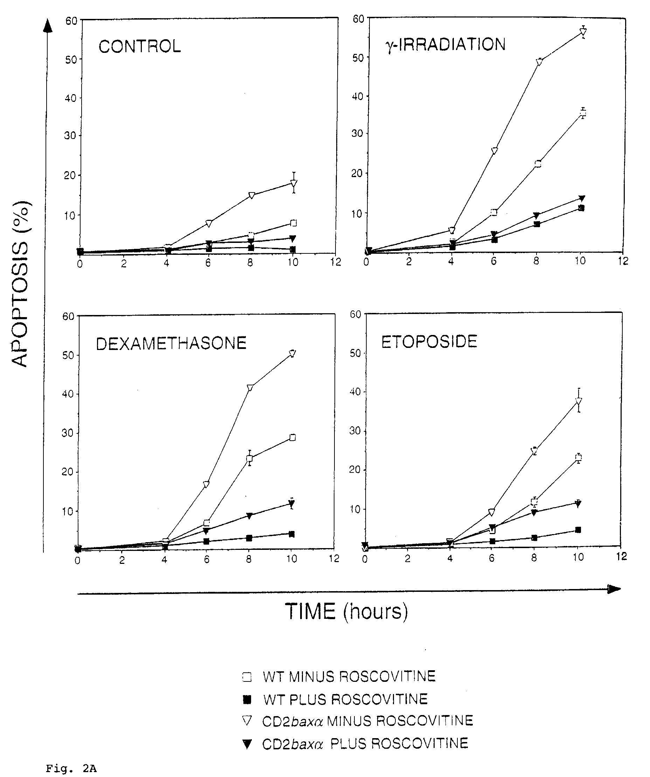

Roscovitine was developed as a competitive analogue of ATP which selectively inhibits Cdk kinase activity with specificity for Cdkl (Cdc2), Cdk2 and Cdk5. We analysed the consequences for thymocyte apoptosis of incubation with roscovitine. Thymocyte suspensions from wild type and baxα transgenic mice were treated as above with dexamethasone, γ-radiation as well as 5OµM etoposide (a DNA topoisomerase II inhibitor used in chemotherapy) in the presence of 25µM roscovitine or an equivalent volume of DMSO used as a vehicle. As shown in Figure 2a, roscovitine was able to block both p53 independent (dexamethasone) and p53 dependent (γ-radiation and etoposide) apoptosis in thymocytes from wild type mice.

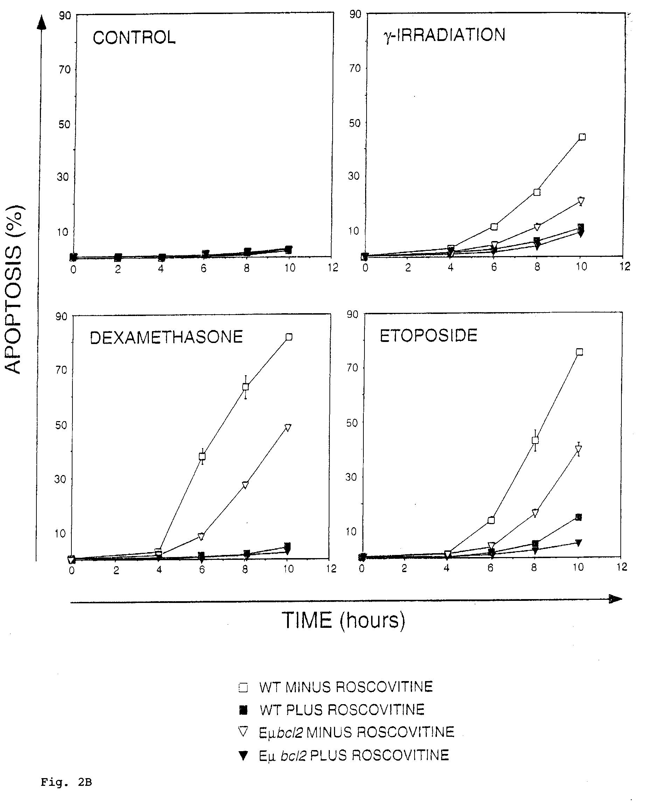

Roscovitine was also able to inhibit Baxα accelerated apoptosis in transgenic thymocytes. Similar experiments showed that 25µM roscovitine could also inhibit the residual apoptosis found in bcl-2 transgenic thymocytes as compared to wild type controls (Fig. 2b). However, not all apoptotic stimuli were blocked by roscovitine.

Fas induced apoptosis in thymocytes was insensitive to the presence of roscovitine, with or without cycloheximide (Fig. 2c). Fas/APO-1 is a cell surface protein that induces apoptosis in immature thymocytes treated with an anti-Fas antibody. Fas induced apoptosis does not require protein synthesis and is accentuated by the presence of cycloheximide. The lack of inhibition by roscovitine on Fas induced apoptosis is consistent with other data, including our own, that shows Bax/Bcl-2 mediated and Fas induced apoptosis of primary T cells to be separate and distinct pathways.

Roscovitine can inhibit Cdk5 activity but since Cdk5 kinase activity has only been detected in neuronal cells this cannot be involved in thymocyte apoptosis.

Roscovitine can also inhibit Cdkl and Cdk2 therefore, to show definitively that Cdk2, as suggested from Figure 1, is the active component required for thymocyte apoptosis we also assayed Cdkl activity during thymocyte apoptosis. It has been suggested that Cdkl plays an important role in triggering apoptosis in a particular cell line though that conclusion has since been disputed. We found that Cdkl activity does not rise as thymocytes undergo apoptosis (data not shown). Thus, the ability of roscovitine to inhibit Cdk2 activity allows it to inhibit the effect of certain apoptotic stimuli.

### Cdk2 activity in apoptosis is not associated with canonical cyclins

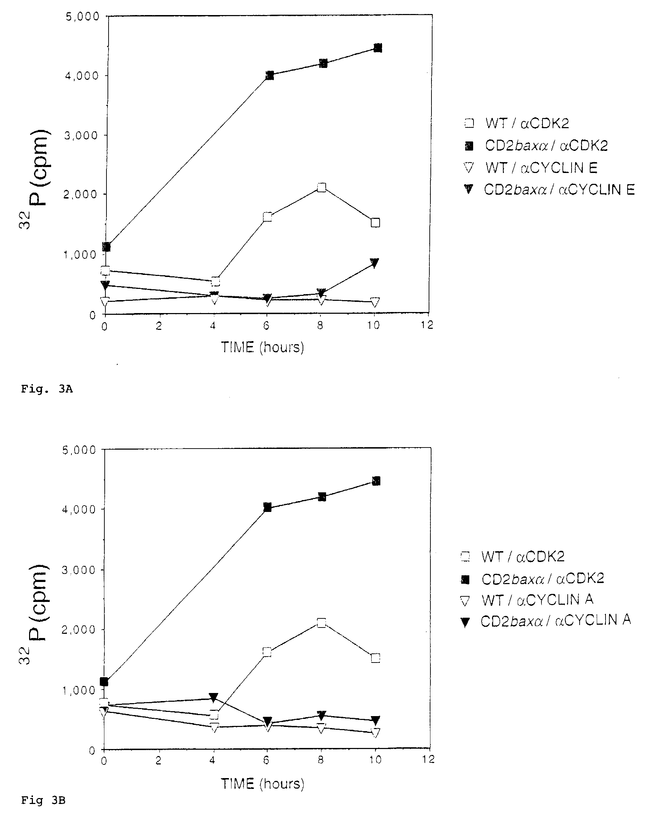

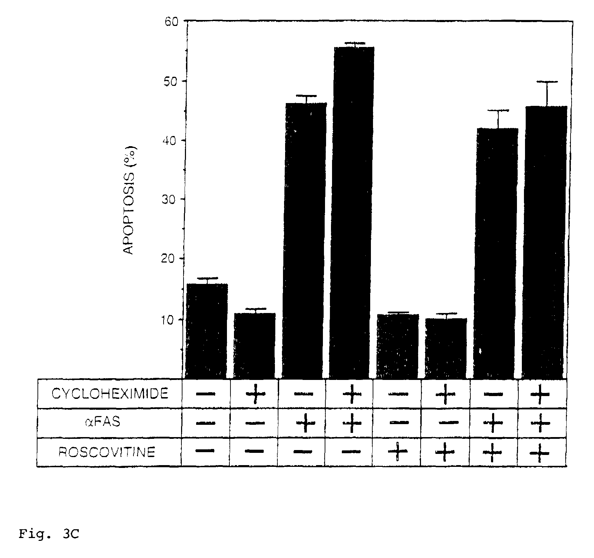

During the cell cycle Cdk2 activity is regulated by association with a cyclin subunit namely, cyclin E then cyclin A. We investigated whether the Cdk2 kinase activity present in apoptosis, thymocytes was associated with either cyclin. We first measured the levels of cyclin E/A for the apoptosis time course shown in Figure 1a. Cyclin E levels were low but remained constant throughout and cyclin A levels were so low throughout as to be virtually undetectable by immunoblot (data not shown). To assess more explicitly whether the Cdk2 kinase activity in dying cells was associated with the canonical cyclins, we measured Cdk2 kinase activity during γ-radiation induced apoptosis in wild type and *baxα* transgenic thymocytes after immunoprecipitation of cychn E and cyclin A with antibodies known to precipitate the active kinase. These values were compared with the kinase activities measured after immunoprecipitation of Cdk2 (Fig. 3a,b). Cyclin E immunoprecipitations lead to the recovery of kinase activities close to background levels constant throughout the time course. Cyclin A associated kinase activities yielded slightly higher values at zero time that gradually declined to background levels after six hours. These values range from three to 10 times less than the corresponding values measured after Cdk2 immunoprecipitation. These experiments strongly suggest that cyclin E and A are not responsible for Cdk2 activation during the apoptotic process. The cyclin A associated kinase activity at zero time is most likely due to the small fraction of proliferating thymocytes present at that time. Our results are compatible with the existence of a so far uncharacterised cyclin-like protein (with name such as roscocyclin or cyclin x) responsible for Cdk2 activation during apoptotic processes. This subunit would need to be synthesised *de novo* or in some way activated in cells triggered to die, thus making the process transcription/translation dependent which is the case for thymocyte apoptosis. The existence of such a subunit would account for the difference in the roles of Cdk2 acting as cell cycle regulator (associating with the "cell division" cyclins E and A) and Cdk2 acting in the apoptotic process associating with an "apoptosis" cyclin.

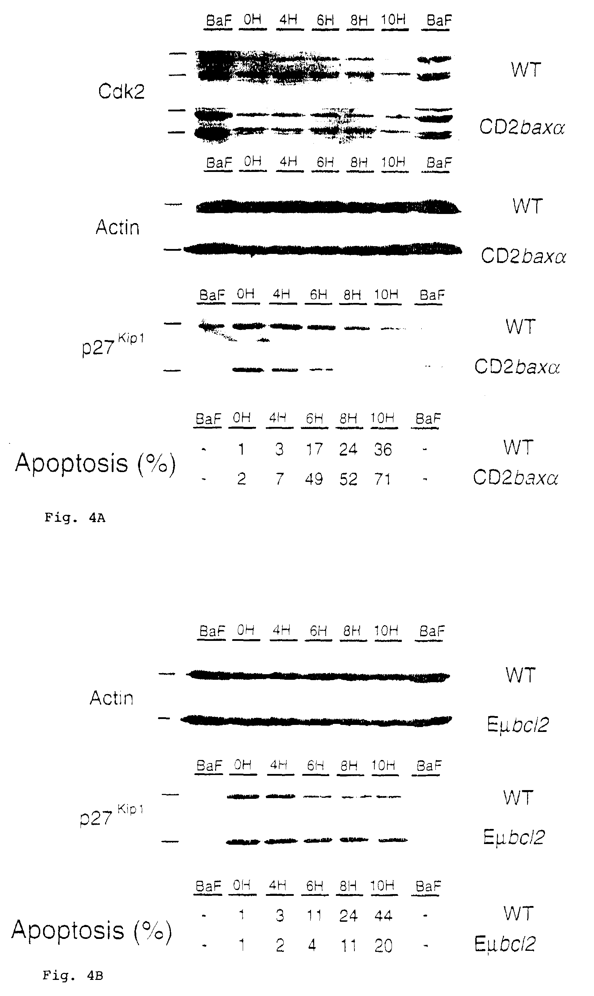

An alternative explanation for the rise in Cdk2 activity during apoptosis could be that the absolute abundance of the protein, rather than its specific activity, is altered. Therefore, to discount this we measured the abundance of Cdk2 during the apoptosis time course by western blot. The amount of Cdk2 protein present did not change appreciably during the apoptosis of γ-irradiated baxa transgenic thymocytes which give high levels of Cdk2 kinase activity (Fig. 4a).

### P27^{Kipl} levels during apoptosis

The activation of Cdk2 during cell cycle by reason of its complexing to cyclins is negatively regulated by p27^{KiPl} 16,25. The fact, that Baxα or Bcl-2 overexpression in T cells is able to modulate p27^{Kipl} levels makes it a suitable candidate for an essential role in the regulation of Cdk2 activity during apoptosis. It was important therefore, to establish that P27^{Kipl} levels decline with the onset of apoptosis. Western blot analysis of wild type and baxa thymocytes following γ-irradiation shows that there is a time-dependent degradation of p27^{KiP1} (Fig. 4a,b) that correlates with Cdk2 activation and the onset of apoptosis (Fig. 1a). As seen with P27^{KiP1} levels during the IL-2 driven G₁-S progression in activated T cells, *bax* overexpression in thymocytes is able to accelerate p27^{Kipl} degradation (Fig. 4a) while the opposite is true for *bcl-2* (Fig. 4b). This result is surprising from the point of view of p27Kip I as a cell cycle brake that needs to be degraded to bring cells into S phase, given that about 90% of the initial population of thymocytes is in the G₀/G₁ state and do not enter S phase during apoptosis. This suggests that the function of P27^{KiP1} is not restricted to its role as cell cycle regulator and can also regulate apoptotic processes.

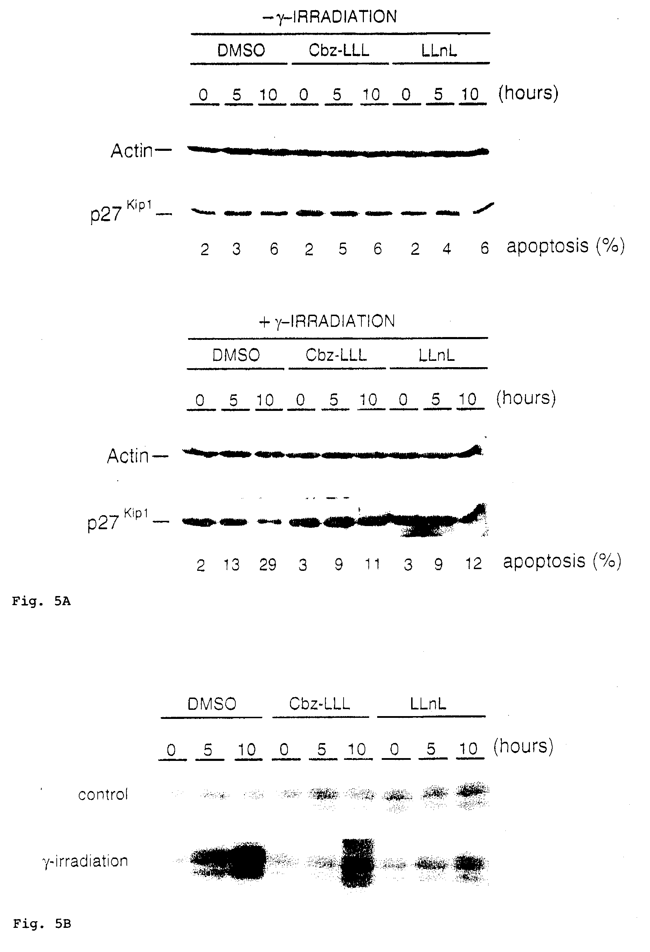

The abundance of P27^{KiP1} is cell cycle regulated by its degradation via the ubiqutin-proteasome pathway, probably during S phase. It has recently been proven that inhibitors of the proteasome are able to block apoptosis in thymocytes and post mitotic neurones. In thymocytes such inhibitors are only effective if added up until five hours after the apoptotic Stimulus. This timing is coincident with the beginning of appreciable p27^{Kip1} degradation in our system (Fig 4a,b). We repeated these experiments by irradiating thymocytes and adding the proteasome inhibitors Cbz-LLL and LLnL, which lead to a substantial inhibition in apoptosis as reported, then we measured the level of p27^{KiP1}. Figure 5 indicates that the presence of the proteasome inhibitors correlates with a decline in the level of p27^{KiP1} degradation (Fig 5a) concomitant with a large increase in the level of Cdk2 kinase activity and hence apoptosis (Fig 5b). This is a further direct indication of the effect of P27^{Kipl} regulated Cdk2 kinase on the progression to cell death.

### Figure Legends

FIG.1 Cdk2 kinase activity during apoptosis. Thymocytes from wild type mice (5 x 10⁵ cells/time point), *baxα* (a), *bcl-2* (b), p53(+/-) and p53(-/-) (c) mice were plated out either without any treatment, after γ-irradiation (5Gy) or in the presence of 2µM dexamethasone. The numbers at the bottom of each line indicate the percentage of apoptotic cells at the corresponding time point. As a positive control (BaF,), 2 x 10⁵ exponentially growing BaF3 cells were processed in the same way as the thymocyte samples for Cdk2 kinase assay. As a negative control (BaF + Block), a similar amount of cell extract was immunoprecipitated in the presence of an excess of the M2 peptide. All the time courses were repeated twice and a representative experiment is shown. The relative intensity of the radioactive bands between two separate treatments can vary slightly due to different autoradiographic exposure times.
FIG.2 Roscovitine inhibition of apoptosis. Thymocytes from wild type and *baxα* (a) or *bcl-2* (b) transgenic mice were processed as indicated in Fig.1 in the presence or absence of 25µM roscovitine. Aliquots (5 x 10⁵ cells) were taken in duplicate at the times indicated and the percentage of cells in apoptosis determined. The mean values and mean deviation are shown. The anti-Fas antibody was used at 1µg/ml and the cycloheximide at 30µg/ml, for an incubation period of 18 hr (c). The experiments were repeated on at least three separate occasions and a representative experiment is shown.
FIG.3 Cyclin E and A associated kinase activity during apoptosis. Wild type and baxa transgenic thymocytes were treated with 5Gy of γ-radiation. Aliquots (5 x 10⁵ cells) were withdrawn at the indicated times and processed for histone Hl kinase activity after immunoprecipitation of cyclin A (a) or cyclin E (b). Radioactive bands were excised from the gel and counted by liquid scintillation. As a positive control, an extract from exponentially growing BaF3 cells was used (c). These experiments were repeated on three separate occasions and a representative experiment is shown.
FIG.4 Cdk2 and P27^{Kipl} levels during apoptosis. *Baxα* (a) and *bcl-2* (b) transgenic thymocytes and thymocytes from wild type littermate controls were treated with 5Gy of γ-radiation then put in culture. Aliquots (5 x 10⁵ cells) were taken at the times indicated and analysed by western blot using antibodies against Cdk2 (a) or against p27^{KiP1} (a,b). As a loading control blots were also probed with a pan-actin antibody. 5 x 10⁵ exponentially growing BaF3 cells were processed as thymocytes and used as a positive control. The percentage apoptosis was determined for duplicate aliquots of the thymocytes used in the western blots.
FIG.5 Effect of proteasome inhibitors on p27^{KiP1} levels, Cdk2 kinase activity and apoptosis. Thymocytes from wild type mice treated with 5Gy of γ-radiation then incubated in the presence or absence of 2µM Cbz-LLL or 5OHM LLNL. Aliquots were withdrawn at the times indicated and the levels of P27^{KiP1} (a) and Cdk2 kinase activity (b) determined.

## Claims

1. Method for inhibiting apoptotic processes in a cell comprising providing said cell with at least one inhibitor of cyclin-dependent kinase 2 or an inhibitor of a functional equivalent of cyclin-dependent kinase 2.

2. Method for inhibiting apoptotic processes in a cell comprising inhibiting cyclin-dependent kinase 2 activity or a functional equivalent of cyclin-dependent kinase 2.

3. Method according to claim 1 or 2 whereby a cell is a T-cell or a cell of neuronal origin or a transplant cell.

4. Roscovitine or a functional equivalent thereof for use in the treatment of diseases associated with apoptosis.

5. Use of roscovitine or a functional equivalent thereof in the preparation of a pharmaceutical for the treatment of traumas involving apoptotic cell death.

6. Use according to claim 4 in the treatment of brain infarct, seizures, heart attacks and the like.

7. Roscovitine or a functional equivalent thereof for use in the treatment of a transplant.

8. Use of roscovitine or a functional equivalent thereof in the preparation of a compound for the treatment of a transplant.

9. Method for inhibiting the cell cycle of a cell, comprising inhibiting interaction between cyclin-dependent kinase 2 or a functional equivalent thereof and the corresponding cyclin.

10. A method for inducing apoptosis in a cell comprising providing said cell with increased cyclin-dependent kinase 2 activity.

11. A means for inducing apoptosis in a subset of cells, comprising a conjugate of a molecule having cyclin-dependent kinase 2 activity and a molecule capable of binding to a target molecule specifically associated with said subset of cells, whereby the conjugate is capable of entering a cell of said subset of cells.

12. A means according to claim 11, further comprising a means for inhibiting the cell cycle stimulating activity of the molecule having cyclin-dependent kinase 2 activity.

13. A means for inhibiting apoptosis in a subset of cells, comprising a molecule inhibiting the apoptotic activity of cyclin-dependent kinase 2 and a molecule capable of binding to a target molecule specifically associated with said subset of cells, whereby the conjugate is capable of entering a cell of said subset of cells.

14. A means according to claim 13, whereby a molecule inhibiting the apoptotic activity is roscovitine or a functional equivalent thereof.

15. A means according to anyone of claims 11-14 whereby the molecule capable of binding to a target molecule specifically associated with said subset of cells is an antibody or a functional equivalent thereof.

16. A means according to anyone of claims 11-14 whereby the target molecule is a receptor.

17. A method for inhibiting apoptosis according to claim 1-3, whereby the inhibitory activity is provided by roscovitine or a functional equivalent thereof or by P27^{kipI} or a functional equivalent thereof.
